# EUROPEAN PATENT SPECIFICATION

(11) **EP 2 258 261 B1**
(45) Date of publication and mention of the grant of the patent: **22.08.2012**
(21) Application number: 10005525.0
(22) Date of filing: 27.05.2010
(51) Int. Cl.: A61B 5/022

(54) **Blood pressure cuff**
Blutdruckmanschette
Manchette pour pression artérielle

(30) Priority: 03.06.2009 JP 2009133771
(43) Date of publication of application: 08.12.2010
(73) Proprietor: Panasonic Corporation, Osaka 571-8501 (JP)
(72) Inventor: Fumuro, Shinichi, Kadoma-shi, Osaka (JP); Kojima, Takeshi, Kadoma-shi, Osaka (JP); Kanetsuna, Yoshitoshi, Kadoma-shi, Osaka (JP)
(74) Representative: Samson & Partner

(56) References cited:
- EP-A2- 2 002 785
- JP-A- 5 317 273
- JP-A- 2002 360 527
- JP-A- 2009 066 199

## Description

### Field of the Invention

The present invention relates to a blood pressure cuff wrapped around an upper arm or a wrist of a human body to occlude blood flow in blood pressure measurement.

### Background of the Invention

Conventionally, a blood pressure cuff for blocking a blood flow is widely known that is wrapped around an upper arm or a wrist of a human body in blood pressure measurement. Such blood pressure cuff according to the preamble of claim 1, for example, is disclosed in Japanese Patent Application Publication No. 05-317273. The blood pressure cuff includes: a cuff body which is wrapped around a wrist to form a cylindrical shape and has an air bag therein; a connector coupled to the air bag of the cuff body to communicate therebetween; and a coupling axle (coupling pipe) rotatably coupled to a coupling axle supporting part provided in the connector. The coupling axle supporting part of the connector has a center axis extending in a radial direction of the cuff body.

The coupling axle is coaxially inserted into the coupling axle supporting part and is rotatable about the center axis thereof. Thus, when a left or a right wrist is inserted into the cylindrical cuff body, an air supply/exhaust tube (air supply tube) which is coupled to the coupling axle and extended (protruded) from the air bag can be rotated in order not to become obstacles.

In the above conventional example, however, as described above, the coupling axle supporting part of the connector is provided such that its center axis is extended in the radial direction of the cuff body. Further, the coupling axle is coaxially inserted into the coupling axle supporting part and is rotated about the center axis.

Therefore, the coupling axle is protruded in the radial direction of the cuff body from an outer peripheral surface of the cuff body so that the protrusion height of the coupling axle from the outer peripheral surface becomes higher, which makes the volume of the blood pressure cuff larger.

JP 2009 066199 describes a sphygmomanometer cuff which has an air supply tube to send air to an air bag and a tube attachment cylindrical part to connect the air supply tube with the air bag. At the end of the air supply tube, a turnable part is provided by which the air supply tube can turn around the tube attachment cylindrical part. In other words, the air supply tube can turn in any direction on any plane and is not restricted in its rotation plane.

### Summary of the Invention

While the invention is defined in the independent claim 1, further aspects of the invention are set forth in the dependent claims, the drawings and the following description:

In viewing of the above, the invention provides a blood pressure cuff capable of lowering a protrusion height of a coupling axle from an outer peripheral surface of a cuff body.

There is provided a blood pressure cuff, which includes: a cuff body which is wrapped around an arm or wrist of a user to form a substantially cylindrical shape when used; an air bag provided in the cuff body, the air bag being charged with air to occlude blood flow; and an air supply/exhaust tube rotatably coupled to the cuff body at one end thereof to communicate with the air bag, an extension direction thereof from the air bag being changeable. In the blood pressure cuff, the air supply/exhaust tube is provided with a coupling axle at the one end thereof, and the coupling axle has a communicating hole that allows the air supply/exhaust tube to communicate with the air bag.

Further, the cuff body is provided with a tube coupling part protruding in a radial direction of the cylindrical cuff body from an outer peripheral surface thereof, and the tube coupling part has a coupling axle supporting part that rotatably supports the coupling axle. Furthermore, the coupling axle supporting part is adapted to align a center axis of the coupling axle in a direction that is approximately perpendicular to an axial direction of the cuff body and is approximately perpendicular to the protruding direction of the tube coupling part, and to rotate the coupling axle about the center axis of the coupling axle.

With such configuration, the protrusion direction of the coupling axle from an outer peripheral surface of the cuff body can be made approximately perpendicular to the axial direction of the tube coupling part, i.e., the radial direction of the cylindrical cuff body. Therefore, the protrusion height of the coupling axle from the outer peripheral surface of the cuff body is lowered in comparison with a conventional case where a protrusion direction of a coupling axle from an outer peripheral surface of a cuff body is directed to a radial direction of the cuff body, thereby enabling to downsize the blood pressure cuff.

The blood pressure cuff, preferably, further includes an air leak prevention unit for preventing air leak from between the coupling axle supporting part and the coupling axle.

This makes it possible to prevent air from leaking from a gap between the coupling axle supporting part and the coupling axle.

In the blood pressure cuff, the air supply/exhaust tube may include: a tube body; and a coupling member coupling the tube body with the cuff body, wherein the coupling member is formed in an L shape in which respective axial directions of both end portions thereof cross with each other at an approximately right angle and one end of the coupling member is coupled to one end of the tube body. Further, the coupling axle is preferably provided at the other end of the coupling member such that the extension direction of the air supply/exhaust tube is changeable between a first direction and a second direction in the axial direction of the cylindrical cuff body as the coupling axle supported by the coupling axle supporting part is rotated, wherein the first direction and the second direction are in opposite directions to each other.

Preferably, the blood pressure cuff further includes a locking unit for releasably locking the air supply/exhaust tube to the cuff body in each of the first direction and the second direction.

With this configuration, even when any forces are applied to the air supply/exhaust tube in usage, for example, it is possible to reduce a load imposed on a joint between the coupling axle supporting part and the coupling axle of the air supply/exhaust tube, thereby making it hard to leak air from the joint.

The locking unit may include locking recessed parts provided in the vicinity of opposite ends of the cylindrical cuff body in axial direction thereof, between which the coupling member is interposed. Preferably, each of the locking recessed parts is configured to releasably hold a portion of the outer periphery of the tube body.

With such configuration, it is possible to easily lock and release the part of the outer periphery of the tube body. Further, since the locking recessed parts are provided only on the cuff body, the structure of the locking unit can be simplified. Thus, the locking unit can be manufactured at low cost.

It is preferable that the locking unit includes: first locking parts provided in the vicinity of opposite ends of the cylindrical cuff body in the axial direction thereof, between which the coupling member is interposed; and a second locking part provided on the air supply/exhaust tube and releasably locked to the first locking part.

In this case, one of the first locking parts and the second locking part may be made of a magnet, and the other may be made of a material attracted to the magnet.

This makes it possible to lock and release the air supply/exhaust tube to and from the cuff body with ease.

### Brief Description of the Drawings

Fig. 1 is a perspective view schematically showing a blood pressure cuff in accordance with an embodiment of the present invention;
Fig. 2 is an enlarged perspective view of a locking recessed part;
Fig. 3 is an enlarged sectional view of a principal part of a joint between a cuff body and an air supply/exhaust tube;
Fig. 4 is a schematic perspective view of the blood pressure cuff rotated by 180 degrees from the state shown in Fig. 1 with the air supply/exhaust tube rotated about a coupling axle by 180 degrees;
Fig. 5 is a plan view of a locking unit in accordance with another example; and
Fig. 6 is a plan view of a locking unit in accordance with further another embodiment.

### Detailed Description of the Embodiments

Hereinafter, the best mode for carrying out the present invention will be explained in detail with reference to the accompanying drawings which form a part hereof.

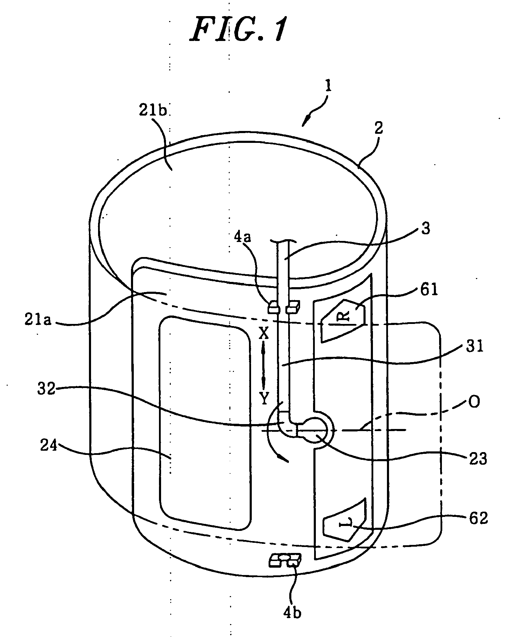

The blood pressure cuff 1 in accordance with an embodiment of the present invention, as shown in Fig. 1, includes: a cuff body 2; an air supply/exhaust tube 3; and locking unit 4a, 4b releasably locking the air supply/exhaust tube 3 to the cuff body 2. In this embodiment, the cuff body 2 is wrapped around an upper arm of a user to form a cylindrical shape for use. Hereinafter, the following explanation will be made based upon the cuff body 2 cylindrically shaped for use.

This cuff body 2 includes:
outer and inner cover fabrics 21a, 21b;
an air bag 22 provided between the outer and inner fabrics 21a and 21b, the air bag 22 serving to occlude blood flow when being charged with the air; and
a connector 23 coupling the air bag 22 and the air supply/exhaust tube 3 together.

The cover fabrics 21a, 21b have a band shape with a certain width in a state that the cuff body is unrolled. Further, the cover fabrics 21a, 21b are wrapped around an upper arm of a user in such a way that the outer cover fabric 21a is arranged at the outside and the inner cover fabric 21b is arranged at the inside.

At one end portion of the outer cover fabric 21a, a first fastener 24 is provided. At a portion of the inner cover fabric 21b facing the first fastener 24 when the cuff body is wrapped, a second fastener (not shown) to be detachably fixed with the first fastener 24 is provided.

When the cuff body 2 is, for example, wrapped around the upper arm, the first fastener 24 is fixed to the second fastener and, accordingly, the cuff body 2 can remain wrapped on the upper arm.

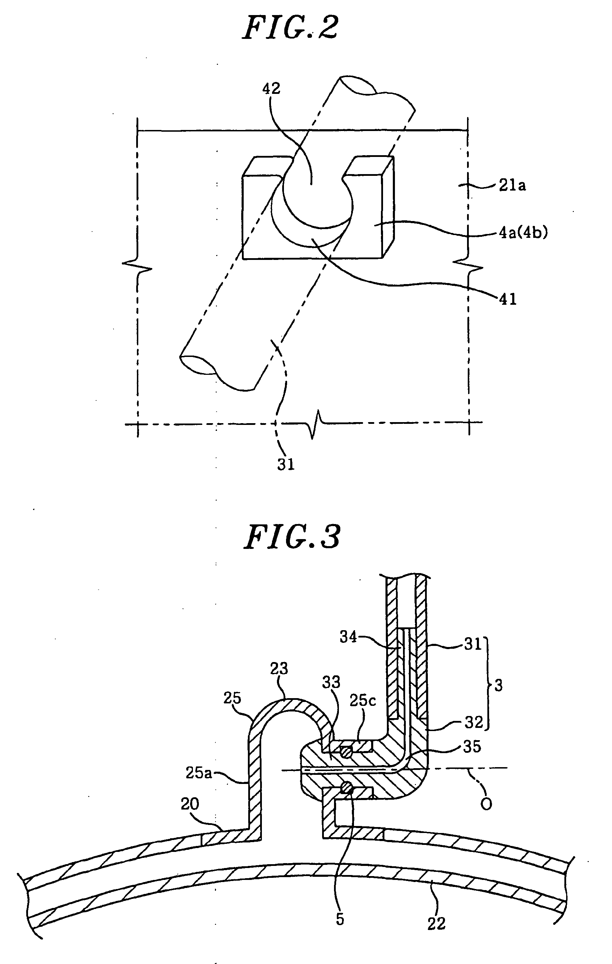

The connector 23, as shown in Fig. 3, includes: an air bag connecting part 20 connected to the air bag 22; and a cylindrical coupling part 25 protruded outwardly from the air bag connecting part 20 in the radial direction of the cuff body and coupled to the air supply/exhaust tube 3.

In the present embodiment, the tube coupling part 25 includes a cylindrical coupling axle supporting part 25c protruding from a peripheral wall 25a thereof in a circumferential direction of the cylindrical cuff body. The coupling axle supporting part 25c is formed in such a way that its axial direction is approximately perpendicular to an axial direction of the tube coupling part 25, i.e., the radial direction of the cuff body and also approximately perpendicular to the axial direction of the cuff body.

Further, as shown in Fig. 1, the shoulder indicating parts 61, 62 are provided in the vicinity of opposite ends of the cuff body 2 in the axial direction thereof. When the cuff body 2 is wrapped around a left arm or a right arm, the respective shoulder indicating parts 61, 62 denote a direction in which the axial direction of the cuff body 2 is to be directed with respect to the left shoulder or the right shoulder.

In the present embodiment, the shoulder indicating parts 61, 62 are configured by the printed character of "R" (in an upward arrow in Fig. 1) and the printed character of "L" (in a downward arrow in Fig. 1).

The air supply/exhaust tube 3 includes: a long tube body 31 which is elastic; and an L-shaped coupling member 32 coupling the tube body 31 to the connector 23. The coupling member 32 is provided with a communicating hole 35 extending therethrough from one end to the other end thereof.

At the one end of the coupling member 32, there is provided a coupling axle 33 coupled to the connector 23, and at the other end thereof, there is provided a tube body coupling part 34 coupled to the one end of the tube body 31, in which the axial direction of the tube body coupling part 34 is perpendicular to that of the coupling axle 33.

The coupling axle 33 is rotatably inserted into the coupling axle supporting part 25c of the connector 23. Accordingly, the coupling axle 33 is coupled to the connector 23 to communicate with the air bag 22. In this coupling state, the center axis "O" of the coupling axle 33 coincides with the center axis of the coupling axle supporting part 25c, and the coupling axle 33 is rotatably supported at the coupling axle supporting part 25c about the center axis "O".

In this embodiment, the coupling axle 33 is configured to have an outer diameter slightly larger than an inner diameter of the coupling axle supporting part 25c, and the coupling axle 33 is forcedly pushed into an inner periphery of the coupling axle supporting part 25c.

With such configuration, after the coupling axle 33 is inserted into the inner periphery of the coupling axle supporting part 25c, it becomes hard to generate a gap between the outer periphery of the coupling axle 33 and the inner periphery of the coupling axle supporting part 25c, which prevents the air leakage therefrom. For example, even if other force than the force of rotating the coupling axle 33 is applied to the coupling axle supporting part 25c, such configuration makes it difficult to generate a gap between the outer periphery of the coupling axle 33 and the inner periphery of the coupling axle supporting part 25c, thereby preventing the air leakage therefrom.

In this embodiment, between the coupling axle 33 forcedly pushed into the interior of the coupling axle supporting part 25c, and the coupling axle supporting part 25c, there is disposed a rotation resistance reducing unit, which is a lubricant such as a grease for reducing the rotation resistance between the coupling axle 33 and the coupling axle supporting part 25c.

As described above, in the case where the coupling axle 33 has an outer diameter larger than an inner diameter of the coupling axle supporting part 25c, the rotation resistance between the coupling axle 33 and the coupling axle supporting part 25c becomes larger. However, the lubricant can reduce the rotation resistance, thereby making it easy to rotate the coupling axle 33.

Furthermore, in this embodiment, there is provided an O-ring 5 made of a synthetic rubber at the outer periphery of the coupling axle 33. Therefore, it is possible to prevent the air from leaking from between the outer periphery of the coupling axle 33 and the inner periphery of the coupling axle supporting part 25c.

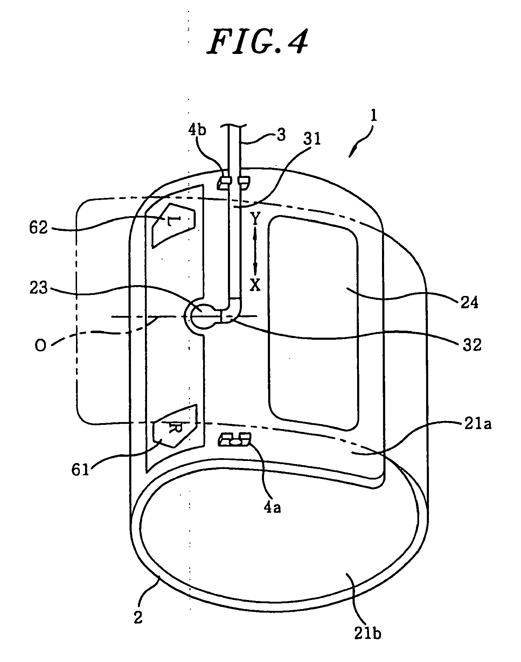

The tube body coupling part 34 is coupled to one end of the tube body 31. Thus, the extension direction of the air supply/exhaust tube 3 is allowed to be changed from a first direction to a second direction as the coupling axle 33 is rotated. For example, the extension direction of the air supply/exhaust tube 3 can be changed from the first direction (X direction) directed to one end in the axial direction of the cuff body as shown in Fig. 1, to the second direction (Y direction) directed to the other end in the axial direction of the cuff body as shown in Fig. 4.

Further, the other end of the tube body 31 is coupled to, e.g., an air pump (not shown) by which air supplied to and exhausted from the air bag, via the air supply/exhaust tube 3.

Next, a locking unit will be explained. As shown in Fig. 1, the locking unit of this embodiment is constituted by locking recessed parts 4a, 4b, which are disposed in the vicinity of opposite ends in the axial direction of the cylindrical cuff body 2 on the outer peripheral surface thereof, the coupling member 32 being interposed between the locking recessed parts 4a, 4b.

The locking recessed part 4a or 4b is used when the cuff body 2 is wrapped around the left or right arm, respectively. The locking recessed part 4a for a left arm, for instance, locks the tube body 31 of the air supply/exhaust tube 3 when the cuff body 2 is wrapped around the left arm. On the other hand, the locking recessed part 4b for a right arm, for instance, locks the tube body 31 of the air supply/exhaust tube 3 when the cuff body 2 is wrapped around the right arm.

Since the locking recessed part 4a for a left arm and the locking recessed part 4b for a right arm have an identical configuration, hereinafter the locking recess 4a for a left arm is explained with reference to Fig. 2 and an explanation of the locking recessed part 4b for a right arm will be omitted.

The locking recessed part 4a for a left arm includes: a recess 41 having a circular arc-shaped cross section for receiving a part of the tube body 31; and an opening 42 through which the part of the tube body is received in and released from the recess 41. The recess 41 is configured to have an inner diameter substantially the same as the outer diameter of the tube body 31.

On the other hand, the opening 42 is configured to have an opening width slightly smaller than the outer diameter of the tube body 31. When being pushed into the opening 42, the tube body 31 is inserted into the recess 41 to be locked by its own elasticity.

Next, an operation of the blood pressure cuff 1 in accordance with the embodiment of the present invention will be explained. First of all, when the blood pressure cuff 1 is used to be wrapped around the left arm of a user, the tube body 31 is locked in the locking recessed part 4a for a left arm as shown in Fig. 1.

The axial direction of the cuff body is aligned such that the left shoulder indicating part 62 is directed toward a user. According to the indication of the left shoulder indicating part 62, the cuff body 2 is wrapped around the upper left arm such that the left shoulder indicating part 62 is directed to the left shoulder of the user, and then the first fastener 24 and the second fastener are fixed without looseness.

In this case, since the extension direction of the air supply/exhaust tube 3 is directed to the left wrist side, the cuff body 2 is allowed to be wrapped without being bothered by the air supply/exhaust tube 3 and others. Further, the user can easily wind the cuff body 2 by gripping its tip end with his/her right hand.

In this state, when air is supplied into the air bag 22 to inflate the air bag 22, the air bag 22 presses arteries passing along the upper arm to occlude blood flow. Subsequently, the blood pressure measurement is performed while the air is gradually exhausted from the air bag 22 and the air bag 22 is deflated.

In the measurement of blood pressure, even if the operator (i.e. , user) hits the tube body 31 with his/her hand or the like, the force applied to the tube body 31 is absorbed by the locking recessed part 4a for a left arm because the tube body 31 is locked in the locking recessed part 4a for a left arm. Accordingly, the force is hardly applied to a joint between the coupling axle 33 and the coupling axle supporting part 25c of the connector 23, and thus the air leak from the joint can be prevented.

Further, when the cuff body 2 is wrapped around the upper arm in a state that the coupling axle supporting part 25c is turned upwardly, the air supply/exhaust tube 3 is allowed to be rotated in a vertical plane. Therefore, the extension direction of the air supply/exhaust tube 3 can be easily changed toward the wrist side only by rotating the air supply/exhaust tube 3 in the vertical plane. On the contrary, in the conventional cuff, the air supply/exhaust tube 3 rotates about an axis extending in the radial direction of the cuff body 2 in a horizontal plane. Therefore, there is a need to give some attention in order to adjust the air supply/exhaust tube to be extended to the wrist side. However, such disadvantages can be eliminated by the present embodiment.

Moreover, once the air supply/exhaust tube 3 is adjusted to head for the wrist side and fixed by the locking unit, the air supply/exhaust tube 3 is hardly rotated horizontally even if an operator accidentally hits the air supply/exhaust tube 3 with his/her hand or the like. This makes it easier to keep the air supply/exhaust tube 3 directed to the wrist side.

On the other hand, in the case where the cuff body 2 is wrapped around the right upper arm from the state shown in Fig. 1, the following operations are carried out. As shown in Fig. 4, the axial direction of the cuff body 2 is changed by 180 degrees in such a way that the right shoulder indicating part 61 is arranged to head for the user. Then, the tube body 31 is released from the locking recessed part 4a for a left arm, the coupling axle 33 of the air supply/exhaust tube 3 is rotated by 180 degrees, and then the tube body 31 is forcedly pushed into the locking recessed part 4b for a right arm to be locked thereby.

Subsequently, the same operation as the case where the cuff body 2 is wrapped around the left arm is carried out. Also in this case, since the extension direction of the air supply/exhaust tube 3 is directed to the right wrist side, the cuff body 2 can be wrapped around the right upper arm without being bothered by the air supply/exhaust tube 3 and the like. Further, the user can easily wind the cuff body 2 by gripping its tip end with his/her right hand.

In the blood pressure measurement, even if an operator (i.e., user) hits the tube body 31 with his/her hand or the like, the force applied to the tube body 31 is absorbed by the locking recessed part 4b because the tube body 31 is locked in the locking recessed part 4b for a right arm. Accordingly, the force is hardly applied to the joint between the coupling axle 33 and the coupling axle supporting part 25c of the connector 23, and thus the air leak from the joint can be prevented.

In the above embodiment, the locking unit is constituted by the locking recessed parts 4a, 4b attached to the cuff body 2, but the locking unit is not limited to this configuration and may be properly modified.

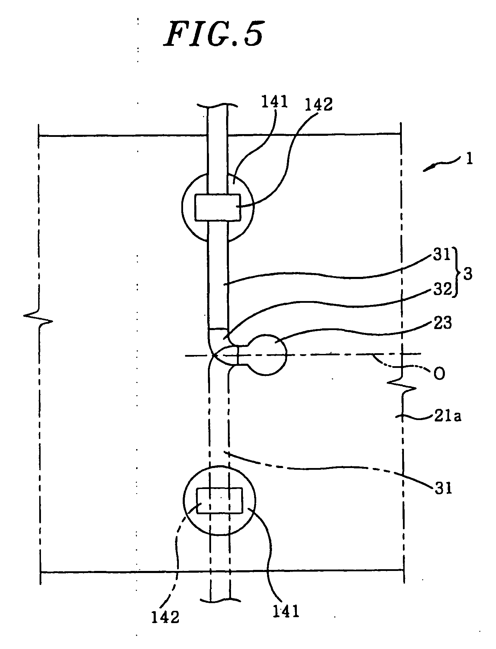

For instance, as shown in Fig. 5, a locking unit is configured to include: first locking parts 141 provided in the cuff body 2; and a second locking part 142 provided in the air supply/exhaust tube 3 to be releasably locked to the first locking part 141.

More specifically, the first locking parts 141 are disposed in the vicinity of opposite ends of the cuff body 2 in the axial direction thereof, between which the coupling member 32 is interposed. The second locking part 142 is disposed at a portion corresponding to the first locking parts 141 at the tube body 31 of the air supply/exhaust tube 3. One of the first locking part 141 and the second locking part 142 is made of a magnet and the other is made of a material attracted to the magnet, e.g., a magnet and iron.

Thus, the extension direction of the air supply/exhaust tube 3 may be locked to each of the opposite positions; i.e., a first position where the air supply/exhaust tube 3 extends to the left wrist side when the cuff body 2 is wrapped around the left arm (the air supply/exhaust tube 3 is directed to the right shoulder side when the cuff body 2 is wrapped around the right arm), and a second position where the air supply/exhaust tube 3 is rotated by 180 degrees from the first position and extends to the left shoulder as illustrated by the dashed line in Fig. 5 (i.e., the position where the air supply/exhaust tube 3 is directed to the right wrist side when the cuff body 2 is wrapped around the right arm).

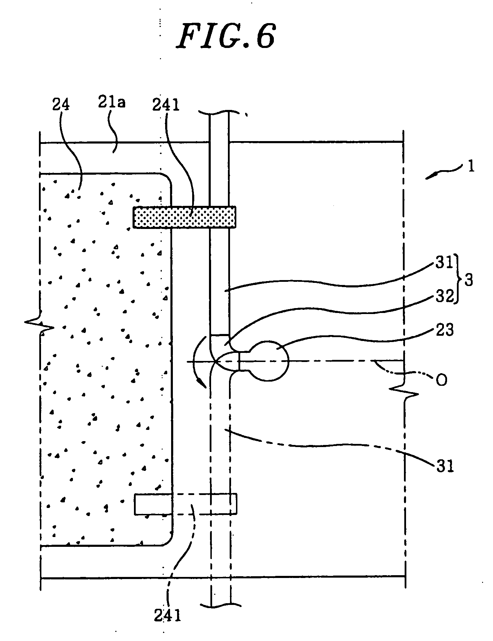

Alternatively, as shown in Fig. 6, the first planar fastener 24 may be provided on the outer cover fabric 21a as the first locking part described above, and, as the second locking part, a locking piece 241 may be attached to the air supply/exhaust tube 3. On both surface sides of the locking piece 241, there are preferably provided planar fasteners to be detachably fixed to the first fastener 24.

In the above embodiment, as the air leak prevention unit, the O-ring 5 is provided between the outer periphery of the coupling axle 33 and the inner periphery of the coupling axle supporting part 25c, while the coupling axle 33 is configured to have the outer diameter slightly larger than the inner diameter of the coupling axle supporting part 25c. However, the air leak prevention unit is not limited to this configuration and may be appropriately modified.

For instance, instead of the air leak prevention unit, the coupling axle 33 may be configured to have an outer diameter slightly larger than the inner diameter of the coupling axle supporting part 25c without disposing the O-ring 5. Alternatively, the coupling axle 33 may be provided whose outer diameter is slightly smaller than the inner diameter of the coupling axle supporting part 25c and the O-ring 5 may be disposed between the couple axle 33 and the coupling axle supporting part 25c.

In the above embodiment, although being formed to be wrapped around an upper arm of a user, the cuff body 2 is not limited to this configuration, but may be wrapped around, e.g., a wrist and be appropriately modified.

## Claims

1. A blood pressure cuff, comprising:
a cuff body (2) which is adapted to be wrapped around an arm or wrist of a user to form a substantially cylindrical shape when used;
an air bag (22) provided in the cuff body (2), the air bag (22) being adapted for being charged with air to occlude blood flow; and
an air supply/exhaust tube (3) rotatably coupled to the cuff body (2) at one end of the air supply/exhaust tube (3) to communicate with the air bag (22), an extension direction of the air supply/exhaust tube (3) from the air bag (22) being changeable,
wherein the air supply/exhaust tube (3) is provided at the one end thereof with a coupling axle (33) the coupling axle (33) having a communicating hole that allows the air supply/exhaust tube (3) to communicate with the air bag (22),
wherein the cuff body (2) is provided with a tube coupling part (25) protruding in a radial direction from an outer peripheral surface of the cuff body (2) the tube coupling part (25) having a coupling axle supporting part (25c) that rotatably supports the coupling axle (33),
wherein the coupling axle supporting part (25c) is formed in such a way that its axial direction is approximately perpendicular to the radial direction of the cuff body (2) and also approximately perpendicular to the axial direction of the cuff body (2), and
wherein the coupling axle (33) is rotatably inserted into the coupling axle supporting part (25c) and the center axis (O) of the coupling axle (33) coincides with that of the coupling axle supporting part (25c), and the coupling axle (33) is rotatably supported about its center axis (O) at the coupling axle supporting part (25c),
the blood pressure cuff being **characterized in that**
the air supply/exhaust tube (3) includes a long tube body (31) and an L-shapes coupling member (32) provided, at one end thereof, with the coupling axle (33) and at the other end thereof, with a tube body coupling part (34) coupled to one end of the tube body (31), in which the axial direction of the tube body coupling part (34) is perpendicular to that of the coupling axle (33).

2. The blood pressure cuff as set forth in claim 1, further comprising an O-ring (5), wherein the coupling axle (33) is inserted into an inner periphery of the coupling axle supporting part (25c) and the O-ring (5) is provided between an outer periphery of the coupling axle(33) and the inner periphery of the coupling axle supporting part(25c) to prevent air leak therebetween.

3. The blood pressure cuff as set forth in claim 1 or 2,
wherein the extension direction of the air supply/exhaust tube (3) is changeable between a first direction and a second direction as the coupling axle (33) supported by the coupling axle supporting part (25c) is rotated, the first direction and the second direction being in opposite directions to each other in the axial direction of the cylindrical cuff body (2), and
wherein the blood pressure cuff further comprises a locking unit for releasably locking the air supply/exhaust tube (3) to the cuff body (2), in each of the first direction and the second direction.

4. The blood pressure cuff as set forth in claim 3,
wherein the locking unit includes locking recessed parts (4a and 4b) provided in the vicinity of opposite ends of the cylindrical cuff body (2) in the axial direction thereof, the coupling member (32) being interposed therebetween, and
wherein each of the locking recessed parts (4a and 4b) is configured to releasably hold a portion of the outer periphery of the tube body (31).

5. The blood pressure cuff as set forth in claim 3,
wherein the locking unit includes:
first locking parts (141 or 241) provided in the vicinity of opposite sides of the cylindrical cuff body (2) in the axial direction thereof, the coupling member (32) being interposed therebetween; and
a second locking part (142 or 242) provided in the air supply/exhaust tube (3), the second locking part (142 or 242) being releasably locked to the first locking part (141 or 241).

6. The blood pressure cuff as set forth in claim5,
wherein one of the first locking parts (141) and the second locking part (142) is made of a magnet, and the other is made of a material attracted to the magnet.

## Patentansprüche

1. Blutdruckmanschette umfassend:
einen Manschettenkörper (2), der dazu ausgelegt ist, um einen Arm oder ein Handgelenk von einem Nutzer gewickelt zu werden, um eine im Wesentlichen zylindrische Form anzunehmen, wenn er verwendet wird;
einen Luftbeutel (22), der in dem Manschettenkörper (2) vorgesehen ist, wobei der Luftbeutel (22) dazu ausgelegt ist, mit Luft gefüllt zu werden, um einen Blutfluss zu okkludieren; und
ein Luft-Zufuhr/Ablass-Rohr (3), das drehbar mit dem Manschettenkörper (2) an einem Ende von dem Luft-Zufuhr/Ablass-Rohr (3) gekoppelt ist, um mit dem Luftbeutel (22) zu kommunizieren, wobei eine Erstreckungsrichtung von dem Luft-Zufuhr/Ablass-Rohr (3) von dem Luftbeutel (2) veränderbar ist,
wobei das Luft-Zufuhr/Ablass-Rohr (3) an einem Ende davon mit einer Koppelachse (33) vorgesehen ist, wobei die Kopplungsachse (33) eine Verbindungsöffnung umfasst, die es dem Luft-Zufuhr/Ablass-Rohr (3) ermöglicht, mit dem Luftbeutel (22) zu kommunizieren,
wobei der Manschettenkörper (2) mit einem Rohr-Kopplungselement (25) versehen ist, das in eine radiale Richtung von der äußeren Umfangsfläche des Manschettenkörpers (2) hervorsteht, wobei das Rohr-Kopplungselement (25) ein Kopplungsachsen-Halteelement (25c) umfasst, das die Kopplungsachse (33) drehbar lagert,
wobei das Kopplungsachsen-Halteelement (25c) derart ausgebildet ist, dass seine axiale Richtung sich annähernd senkrecht zu der radialen Richtung des Manschettenkörpers (2) und auch annähernd senkrecht zu der axialen Richtung von dem Manschettenkörper (2) erstreckt, und
wobei die Kopplungsachse (33) drehbar in das Kopplungsachsen-Halteelement (25c) eingeführt ist und die zentrale Achse (O) von der Kopplungsachse (33) mit der von dem Kopplungsachsen-Halteelement (25c) zusammen fällt, und die Kopplungsachse (33) drehbar um seine zentrale Achse (O) an dem Kopplungsachsen-Halteelement (25c) gelagert ist,
die Blutdruckmanschette ist **dadurch gekennzeichnet, dass**
das Luft-Zufuhr/Ablass-Rohr (3) einen langen Rohrkörper (31) und ein L-förmiges Kopplungselement (32) umfasst, die an einem Ende davon mit der Kopplungsachse (33) und an dem anderen Ende davon mit einem Rohrkörper-Kopplungselement (34) vorgesehen sind,, das mit einem Ende von dem Rohrkörper (31) gekoppelt ist, bei dem die axiale Richtung von dem Rohrkörper-Kopplungselement (34) senkrecht zu der von der Kopplungsachse (33) verläuft.

2. Blutdruckmanschette gemäß Anspruch 1, weiter umfassend einen O-Ring (5), wobei die Kopplungsachse (33) in einen inneren Umfang von dem Kopplungsachsen-Halteelement (25c) eingeführt ist und der O-Ring (5) zwischen einem äußeren Umfang von der Kopplungseinachse (33) und dem inneren Umfang von dem Kopplungsachsen-Halteelement (25c) vorgesehen ist, um ein Luftleck dazwischen zu verhindern.

3. Blutdruckmanschette gemäß Anspruch 1 oder 2, bei der die Erstreckungsrichtung von dem Luft-Zufuhr/Ablassrohr (3) zwischen einer ersten Richtung und einer zweiten Richtung veränderbar ist, wenn die Kopplungsachse (33), die über das Kopplungsachsen-Halteelement (25c) gelagert ist, gedreht wird, wobei die erste Richtung und die zweite Richtung zu einander entgegen gesetzte Richtungen sind in der axialen Richtung von dem zylindrischen Manschettenkörper (2), und
wobei die Blutdruckmanschette weiter eine Sperreinheit umfasst, zum freigebbaren Sperren des Luft-Zufuhr/Ablass-Rohres (3) des Manschettenkörpers (2) in jede von der ersten und der zweiten Richtung.

4. Blutdruckmanschette gemäß Anspruch 3, bei der die Sperreinheit sperrende ausgesparte Elemente (4a und 4b) umfasst, die in der Nähe von gegenüberliegenden Enden von dem zylindrischen Manschettenkörper (2) in der axialen Richtung davon vorgesehen sind, wobei das Kopplungselement (32) dazwischen angeordnet ist, und wobei jedes von den sperrenden ausgesparten Elementen (4a und 4b) dazu ausgelegt ist, einen Bereich von dem äußeren Umfang von dem Rohrkörper (31) freigebbar zu halten.

5. Blutdruckmanschette gemäß Anspruch 3,
bei der die Sperreinheit umfasst:
erste Sperrelemente (141 oder 241), die in der Nähe von gegenüber liegenden Seiten von dem zylindrischen Manschettenkörper (2) in der axialen Richtung davon vorgesehen sind, wobei das Kopplungselement (32) dazwischen angeordnet ist; und
ein zweites Sperrelement (142 oder 242), das in dem Luft-Zufuhr/Ablass-Rohr (3) vorgesehen ist, wobei das zweite Sperrelement (142 oder 242) freigebbar an dem ersten Sperrelement (141 oder 241) gesperrt ist.

6. Blutdruckmanschette gemäß Anspruch 5, bei der eines von dem ersten Sperrelementen (141) und dem zweiten Sperrelement (142) aus einem Magnet hergestellt ist und das andere aus einem Material, das von dem Magneten angezogen wird, hergestellt ist.

## Revendications

1. Brassard de prise de tension, comprenant :
un corps de brassard (2) qui est adapté pour être enroulé autour d'un bras ou d'un poignet d'un utilisateur de façon à réaliser une forme sensiblement cylindrique quand il est utilisé ;
un coussin gonflable (22) disposé dans le corps de brassard (2), le coussin gonflable (22) étant adapté pour être rempli d'air de façon à bloquer le flux sanguin ; et
un tube d'alimentation/de sortie de l'air (3) couplé de manière rotative au corps de brassard (2) au niveau d'une extrémité du tube d'alimentation/de sortie de l'air (3) de façon à communiquer avec le coussin gonflable (22), la direction d'extension du tube d'alimentation/ de sortie de l'air (3) à partir du coussin gonflable (22) pouvant être modifiée ;
dans lequel le tube d'alimentation/de sortie de l'air (3) est doté au niveau d'une extrémité de celui-ci d'un axe d'accouplement (33), l'axe d'accouplement (33) présentant un trou de communication qui permet au tube d'alimentation/de sortie de l'air (3) de communiquer avec le coussin gonflable (22) ;
dans lequel le corps de brassard (2) est doté d'une partie d'accouplement de tube (25) qui fait saille dans une direction radiale à partir d'une surface périphérique extérieure du corps de brassard (2), la partie d'accouplement de tube (25) présentant une partie de support d'axe d'accouplement (25c) qui supporte de manière rotative l'axe d'accouplement (33) ;
dans lequel la partie de support d'axe d'accouplement (25c) est formée de telle manière que sa direction axiale soit approximativement perpendiculaire à la direction radiale du corps de brassard (2) et qu'elle soit également approximativement perpendiculaire à la direction axiale du corps de brassard (2) ; et
dans lequel l'axe d'accouplement (33) est inséré de manière rotative dans la partie de support d'axe d'accouplement (25c) et l'axe central (O) de l'axe d'accouplement (33) coïncide avec celui de la partie de support d'axe d'accouplement (25c), et l'axe d'accouplement (33) est supporté de manière rotative autour de son axe central (O) au niveau de la partie de support d'axe d'accouplement (25c) ;
le brassard de prise de tension étant **caractérisé en ce que** :
le tube d'alimentation/de sortie de l'air (3) comprend un corps de tube long (31) et un élément d'accouplement en forme de L (32) doté au niveau d'une extrémité de celui-ci, de l'axe d'accouplement (33) et, au niveau de l'autre extrémité de celui-ci, d'une partie d'accouplement de corps de tube (34) accouplée à une extrémité du corps de tube (31), dans lequel la direction axiale de la partie d'accouplement de corps de tube (34) est perpendiculaire à celle de l'axe d'accouplement (33).

2. Brassard de prise de tension comme déterminé dans la revendication 1, comprenant en outre un joint torique (5), dans lequel l'axe d'accouplement (33) est inséré dans une périphérie intérieure de la partie de support d'axe d'accouplement (25c) et le joint torique (5) est disposé entre une périphérie extérieure de l'axe d'accouplement (33) et la périphérie intérieure de la partie de support d'axe d'accouplement (25c) de façon à empêcher une fuite d'air entre eux.

3. Brassard de prise de tension selon la revendication 1 ou la revendication 2,
dans lequel la direction d'extension du tube d'alimentation/de sortie de l'air (3) peut être modifiée entre une première direction et une seconde direction lorsque l'axe d'accouplement (33) supporté par la partie de support d'axe d'accouplement (25c) est tourné, la première direction et la seconde direction étant des directions opposées l'une par rapport à l'autre dans la direction axiale du corps de brassard cylindrique (2) ; et
dans lequel le brassard de prise de tension comprend en outre une unité de verrouillage destinée à verrouiller le tube d'alimentation/de sortie de l'air (3) avec le corps de brassard (2), dans chacune de la première direction et de la seconde direction.

4. Brassard de prise de tension selon la revendication 3,
dans lequel l'unité de verrouillage comprend des parties renfoncées de verrouillage (4a et 4b) disposées à proximité des extrémités opposées du corps de brassard cylindrique (2) dans la direction axiale de celui-ci, l'élément d'accouplement (32) étant interposé entre ; et
dans lequel chacune des parties renfoncées de verrouillage (4a et 4b) est configurée de façon à tenir de manière libérable une partie de la périphérie extérieure du corps de tube (31).

5. Brassard de prise de tension selon la revendication 3,
dans lequel l'unité de verrouillage comprend :
des premières parties de verrouillage (141 or 241) disposées à proximité des côtés opposés du corps de brassard cylindrique (2) dans la direction axiale de celui-ci, l'élément d'accouplement (32) étant interposé entre ; et
une seconde partie de verrouillage (142 ou 242) disposée dans le tube d'alimentation/de sortie de l'air (3), la seconde partie de verrouillage (142 ou 242) étant verrouillée de manière libérable avec la première partie de verrouillage (141 ou 241).

6. Brassard de prise de tension selon la revendication 5,
dans lequel l'une des premières parties de verrouillage (141) et de la seconde partie de verrouillage (142) est constituée d'un aimant, et l'autre est constituée d'un matériau attiré par l'aimant.
